(19)

Europäisches
Patentamt
European
Patent Office
Office européen
des brevets

(11) **EP 2 918 216 B1**

(12) **EUROPEAN PATENT SPECIFICATION**

(45) Date of publication and mention
of the grant of the patent:
**12.04.2017 Bulletin 2017/15**

(51) Int Cl.:
**A61B 1/00** *(2006.01)*    **A61B 90/35** *(2016.01)*

(21) Application number: **13853422.7**

(86) International application number:
**PCT/JP2013/000738**

(22) Date of filing: **12.02.2013**

(87) International publication number:
**WO 2014/073122 (15.05.2014 Gazette 2014/20)**

(54) **ENDOSCOPE OPERATING SYSTEM**

**ENDOSKOPBETRIEBSSYSTEM**

**SYSTÈME DE FONCTIONNEMENT D'UN ENDOSCOPE**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.11.2012  JP 2012245703**

(43) Date of publication of application:
**16.09.2015  Bulletin 2015/38**

(73) Proprietor: **Tokyo Institute of Technology
Tokyo 152-8550 (JP)**

(72) Inventors:
• **TADANO, Kotaro
  Tokyo 152-8550 (JP)**

• **KAWASHIMA, Kenji
  Tokyo 152-8550 (JP)**

(74) Representative: **Cabinet Laurent & Charras
Le Contemporain
50 Chemin de la Bruyère
69574 Dardilly Cedex (FR)**

(56) References cited:
**EP-A1- 1 596 272        JP-A- H08 332 169
JP-A- H11 104 064      JP-A- 2001 145 634
JP-A- 2009 285 099    JP-A- 2011 194 163
US-A- 5 876 325          US-A1- 2011 234 484**

Printed by Jouve, 75001 PARIS (FR)

**Description**

**Technical Field**

The present invention relates to an endoscope operation system.

**Background Art**

As surgical operations, endoscopic surgeries are widely performed instead of performing abdominal operations because of the advantages of the endoscopic surgeries such as faster recoveries after the operations and smaller surgical incisions. In such endoscopic surgeries, a master-slave type endoscope operation system that enables operations by remote control has been proposed.

In such endoscope operation systems, as shown in Patent Literature 1, for example, the magnification ratio of a zoom lens of an endoscope is controlled based on a detection output from a posture sensor that is disposed in a head-mounted display (hereinafter also called "HMD") of an operating surgeon and detects the movement of the surgeon's head. Further, the movement of the surgeon's head is obtained as the displacement of the posture sensor with respect to a magnetic source that generates a magnetic field. In this way, when the surgeon turns left with respect to the subject (i.e., the patient), for example, a left image created based on picked-up image data obtained through a solid-state image-pickup device of the endoscope is displayed in a pair of liquid crystal monitors disposed inside the HMD. Further, when the surgeon moves closer to the subject, a visual field that is magnified by the zoom lens is obtained. Therefore, the surgeon can observe the inside of a body cavity into which the endoscope is inserted in a 3D (three-dimensional) manner.

Further, Non-patent Literature 1 proposes an endoscope grasping device including a five-joint link mechanism, a ball-joint section for holding a trocar penetrating an abdominal wall in an abdominal wall part, a drive section that drives the link mechanism, and an operation section. In this configuration, a laparoscope, which is a kind of endoscope, is a zoom type and can swiftly switch an image between proximal and distal images. Further, the zoom type laparoscope can be speedily moved to a place the surgeon desires by using a controller switch.

**Citation List**

**Patent Literature**

Patent Literature 1: Japanese Unexamined Patent Application Publication No. H10-309258

**Non Patent Literature**

Non-patent Literature 1: Medical endoscope grasping device "Naviot", catalog, published by Hitachi Hybrid Network Co., Ltd.

US 2011/234484 discloses a prior art device according to the preamble of claim 1.

**Summary of Invention**

**Technical Problem**

In the endoscope operation system disclosed in Non-patent Literature 1 and the like, the zooming operation is performed by using, for example, a voice command or a switch operated by a hand or a foot. Therefore, an operating surgeon needs to learn a zooming operation method and the like in advance. Further, when the surgeon performs a zooming operation during a surgery, he/she needs to change his/her attention from the surgical manipulation to the zooming operation. Therefore, there is a room for an improvement in the working efficiency and a risk of operation errors.

Therefore, it is considered that if the zooming operation can be performed by moving the surgeon's face forward/backward as performed in daily life, it can be performed in a very intuitive manner. To that end, it is necessary to detect the translational motion of the head by using some kind of method. As means for detecting the translational motion of a head, there is a method using an optical or magnetic 3D detection device. However, it is necessary to attach a marker or the like to the head of an operator and fix a sensor to some place external to the operator. Therefore, in addition to a restriction on the positional relation between the external sensor and the operator, there are problems that signals could be blocked and interference with other devices could occur. Meanwhile, it is theoretically possible to attach an acceleration sensor to the head of an operator and calculate the speed of the head by integrating outputs of the acceleration sensor. However, it is very difficult to accurately calculate the speed because of errors in the gravitational acceleration compensation, the deviation of the zero point, noises, and so on.

It should be noted that the endoscope operation system disclosed in Patent Literature 1 adopts a method in which a magnetic sensor is attached to an HMD and the movement of the head of a surgery is detected based on changes in a magnetic field. Therefore, since the endoscope operation system is affected by a magnetic field, the endoscope operation system cannot be practically used under MRI environments. Further, it is assumed that the up/down, left/right, and zooming actions of a curved endoscope are controlled by the HMD.

The present invention has been made to solve the above-described problems and an object thereof is to provide an endoscope operation system capable of easily and intuitively performing a zooming operation of a visual field of an endoscope.

## Solution to Problem

According to an exemplary embodiment, an endoscope operation system includes: an endoscope including an image pickup unit at its tip; a holding arm unit that supports the tip of the endoscope in such a manner that the tip of the endoscope can be moved in up/down, left/right, and front/back directions, and can be rotated on its own axis; a display unit that displays an image based on an image signal supplied from the image pickup unit of the endoscope; a first posture detection unit attached to a head of an operator, the first posture detection unit being configured to detect an angular speed based on a postural displacement of the head of the operator; a second posture detection unit attached to a torso of the operator, the second posture detection unit being configured to detect an angular speed based on an inclinational displacement of an upper body of the operator; and a control unit that calculates a target speed vector at the tip of the endoscope and controls a moving direction and a speed of the tip of the endoscope so that the tip of the endoscope follows the calculated target speed vector, in which the control unit calculates a translational speed of a neck position of the operator in the front/back direction based on an inclinational angular speed component of the upper body in the front/back direction of the angular speed calculated based on the inclinational displacement of the upper body of the operator detected by the second posture detection unit, and calculates the target speed vector based on the calculated translational speed of the neck position of the operator in the front/back direction and information of a movement of a posture of the head of the operator detected by the first posture detection unit.

## Advantageous Effects of Invention

According to the present invention, it is possible to provide an endoscope operation system capable of easily and intuitively performing a zooming operation of a visual field of an endoscope.

## Brief Description of Drawings

Fig. 1 is a schematic view for explaining a characteristic configuration and features according to the present invention;
Fig. 2 schematically shows an overall configuration of an endoscope operation system according to a first exemplary embodiment with an operating surgery;
Fig. 3 shows a holding arm unit used in an example of the endoscope operation system according to the first exemplary embodiment;
Fig. 4 is a block diagram showing an overall configuration of an example of the endoscope operation system according to the first exemplary embodiment;
Fig. 5 is a schematic diagram for explaining detection of an inclinational angular speed of an upper body in a front/back direction according to the first exemplary embodiment; and
Fig. 6 is a schematic diagram for explaining detection of a movement of the whole head in an up/down direction according to a third exemplary embodiment.

## Description of Embodiments

First exemplary embodiment

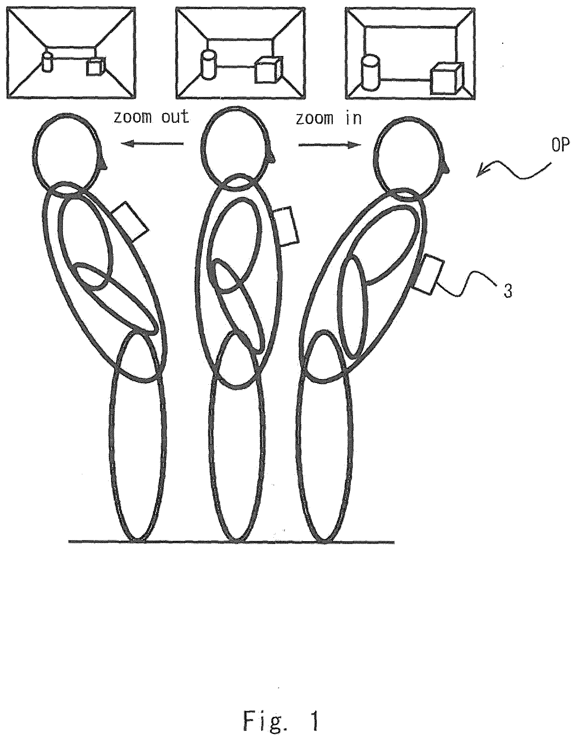

Exemplary embodiments according to the present invention are explained hereinafter with reference to the drawings. A characteristic configuration and features according to the present invention are briefly explained with reference to Fig. 1. An example of an endoscope operation system according to this exemplary embodiment is for intuitively carrying out a translational operation of a visual field such as a zooming operation by moving the head of an operator in the front/back direction in a translational manner as performed in daily life without using his/her hands and feet. In most cases, when a person moves his/her head forward/backward, he/she bends his/her whole upper body. Therefore, instead of directly detecting the translational motion of the head, a posture detection unit such as a gyroscopic sensor is attached to the chest of the operator (operating surgeon OP) and an inclinational angular speed of his/her upper body is detected. Further, a translational operation of a visual field is carried out by using this detection output value.

As shown in Fig. 1, for example, posture detection means such as a gyroscopic sensor 3 is attached to the chest of the surgeon OP and the inclinational angular speed of his/her upper body is detected by using this gyroscopic sensor 3. Then, the translational speed of his/her head in the front/back direction is calculated from the detected upper body inclinational angular speed and the calculated translational speed is used as a command value for a zooming operation or the like. For example, when the upper body is tilted forward, the visual field is zoomed in. Further, when the upper body is tilted backward, the visual field is zoomed out.

To provide an endoscope operation system capable of easily and intuitively performing a zooming operation of a visual field of an endoscope, the inventors of the present application have paid attention to the fact that when a person moves his/her head translationally in the front/back or left/right direction in a natural fashion, he/she bends his/her upper body rather than moving only the head and neck. This motion of the upper body is not a translational motion but is a rotational motion around the waist or the part near the waist. Further, the speed of this rotational motion can be easily detected by attaching a gyroscopic sensor 3 or the like to the upper body. The translational speed of the head in the front/back direction can be calculated from this upper body inclinational angular speed and the calculated translational speed can be used as a command value for a zooming operation and the like. Further, as described later, at least five degrees of freedom of the motion of the head can be detected by combining this calculated translational speed with an output of a gyroscopic sensor attached to the head.

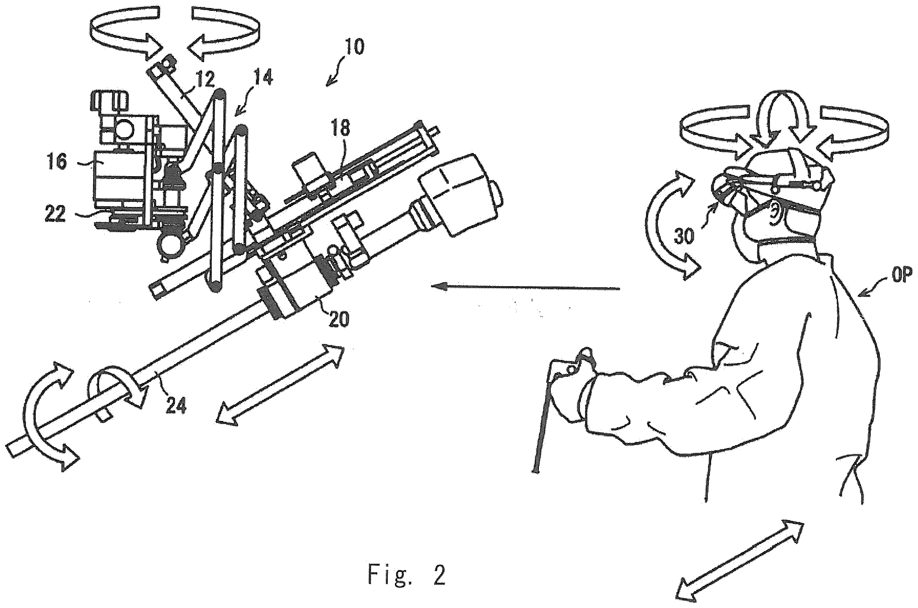

Fig. 2 shows a configuration of an example of the endoscope operation system according to this exemplary embodiment with an operating surgery OP.

In Fig. 2, the endoscope operation system includes as main components, an endoscope 24, a holding arm unit 10 that holds the endoscope 24 and controls the posture of the endoscope 24, and a head-mounted display 30 (hereinafter also called "HMD 30") detachably attached to the head of the surgery OP.

The endoscope 24 includes, for example, an flexible insertion section including an image pickup unit at its tip, which is inserted into a body, an operation unit (not shown) that controls an optical system, and a connection section (not shown) that is connected to the operation unit and connects a light source or the like to the operation unit.

The image pickup unit includes an optical system including an objective lens and so on, a solid-state image pickup device, and a zooming mechanism unit including an actuator that controls a lens(es) of the optical system to scale up or down an image obtained by the image pickup unit. The zooming mechanism unit of the image pickup unit is controlled by an endoscope controller 60, which is described later. A light guide is provided near the objective lens at the tip of the insertion section. The light guide is used to illuminate the inside of the body by light guided from the above-described light source. Note that both a rigid endoscope and a soft endoscope can be used as the endoscope 24.

As shown in Fig. 2, the HMD 30 is attached to the head of the surgery OP. The HMD 30 includes a pair of left and right display units (not shown) in places that directly face the face of the surgery OP and correspond to the respective eyes of the surgery OP. The display units display, for example, a 3D color image. Note that the display units are not limited to the above-shown example. For example, they may display a 2D (two-dimensional) monochrome image.

The whole HMD 30 follows the movement of the head of the surgery OP. That is, for the HMD 30, when viewed from the surgery OP side, movements including a right direction (clockwise) rotation around the central axis line of the neck (right turn), a left direction (counterclockwise) rotation around the central axis line of the neck (left turn), a front/back rotation with respect to the neck (bending, stretching), a tilting movement to the right with respect to the neck (right bending), and a tilting movement to the left with respect to the neck (left bending) can be performed as indicated by double-headed arrows in Fig. 2.

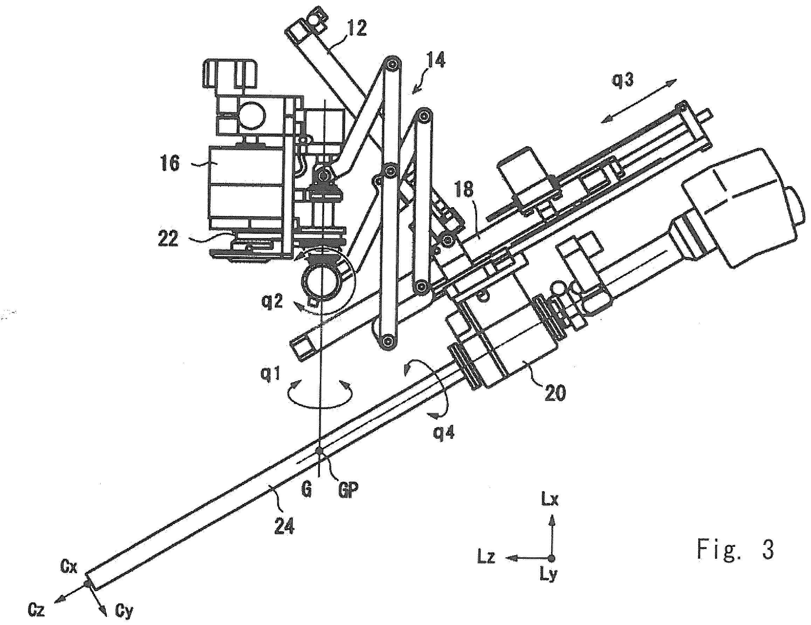

The holding arm unit 10 supports the tip of the endoscope 24 in such a manner that the tip of the endoscope 24 can be moved in up/down, left/right, and front/back directions, and can be rotated on its own axis. The holding arm unit 10 is supported on a pedestal (not shown) located near an operating table located away from the surgery OP through a bracket(s) (not shown) for a vane motor unit 16, which is described later. As shown in Figs. 2 and 3, the holding arm unit 10 includes as main components, a chassis that movably supports a vane motor 20 that rotatably supports the endoscope 24, an air-pressure cylinder 18 that is fixed to the chassis and moves the endoscope 24 and the vane motor 20 closer to or away from a subject (i.e., a patient), a vane motor unit 16 supported on the aforementioned chassis through a parallel linkage 14 whose one end is supported on the chassis, a rotation shaft that is rotated through a timing belt pulley connected to an output shaft of the vane motor unit 16 and a timing belt 22 and thereby rotates the whole chassis, and an air-pressure cylinder 12 that drives the parallel linkage 14.

In the parallel linkage 14, one end of each of link members forming a part of the parallel linkage 14 is connected to the rotation shaft and the other end thereof is connected to the chassis. In this way, when the rod of the air-pressure cylinder 12 connected to the parallel linkage 14 is in an extended state, for example, the chassis is rotated clockwise around the lower end of the rotation shaft in Fig. 3. On the other hand, when the rod of the air-pressure cylinder 12 connected to the parallel linkage 14 is in a contracted state, the chassis is rotated counterclockwise around the lower end of the rotation shaft in Fig. 3. That is, as described later, the image pickup unit of the endoscope 24 can be moved around a rotation center point GP in a direction corresponding to the front/back rotation (bending, stretching) of the head of the surgery OP with respect to his/her neck in the HMD 30. The rotation center point GP is located on a common straight line with a rotation axis line G of the rotation shaft, which is described later, and located near the body wall of the subject. The rotation axis line G is determined in such a manner that the rotation axis line G is in parallel with an Lx-coordinate axis of an orthogonal coordinate system shown in Fig. 3, which is used in the holding arm unit 10. The Lx-coordinate axis is defined in a direction perpendicular to the body wall of the subject, and an Lz-coordinate axis is defined so as to be perpendicular to the Lx-coordinate axis.

The air-pressure cylinder 18 is supported on the chassis so that its rod is roughly in parallel with the central axis line of the endoscope 24. When the rod of the air-pressure cylinder 18 is in an extended state, the image pickup unit of the endoscope 24 and the vane motor 20 are moved with respect to the chassis so that they are moved away from the subject in Fig. 3. On the other hand, when the rod of the air-pressure cylinder 18 is in a contracted state, the image pickup unit of the endoscope 24 and the vane motor 20 are moved with respect to the chassis so that they are moved closer to the subject in Fig. 3. That is, as described later, the image pickup unit of the endoscope 24 can be moved in a direction corresponding to the tilting of the upper body of the surgery OP in the front/back direction.

One end of each of the link members forming the parallel linkage 14 is connected to their respective places that are apart from each other by a predetermined distance along the central axis line in the rotation shaft that is disposed side by side with the vane motor unit 16. The rotation shaft is rotatably supported on the vane motor unit 16 around the rotation axis line G. As a result, when the vane motor unit 16 is in an operating state, the image pickup unit of the endoscope 24 and the vane motor 20 can be rotated around the rotation axis line G. That is, as described later, the image pickup unit of the endoscope 24 can be moved in a direction corresponding to turning of the head of the surgery OP on his/her neck in the HMD 30.

Further, the operation unit and its peripheral part in the endoscope 24 are rotatably supported by the vane motor 20. In this way, the image pickup unit of the endoscope 24 can be rotated on its own axis (roll) around the rotation central axis line of the vane motor 20 within a predetermined angle range. That is, as described later, the image pickup unit of the endoscope 24 can be moved in a direction corresponding to left/right bending of the head of the surgery OP in the HMD 30.

As described above, the image pickup unit of the endoscope 24 can be moved around the rotation center point GP in a direction corresponding to the front/back rotation (bending, stretching) of the head of the surgery OP with respect to his/her neck in the HMD 30. Further, the image pickup unit of the endoscope 24 can be moved in a direction corresponding to turning of the head of the surgery OP on his/her neck in the HMD 30. Further, the image pickup unit of the endoscope 24 can be moved in a direction corresponding to left/right bending of the head of the surgery OP in the HMD 30. Further, the image pickup unit of the endoscope 24 can be moved in a direction corresponding to tilting of the upper body of the surgery OP in the front/back direction. That is, for the movement of the image pickup unit of the endoscope 24, the holding arm unit 10 can carry out at least four degrees of freedom of movements, i.e., movements in the up/down, left/right, rotational, and front/back directions to follow the movement of the head and the upper body of the surgery OP.

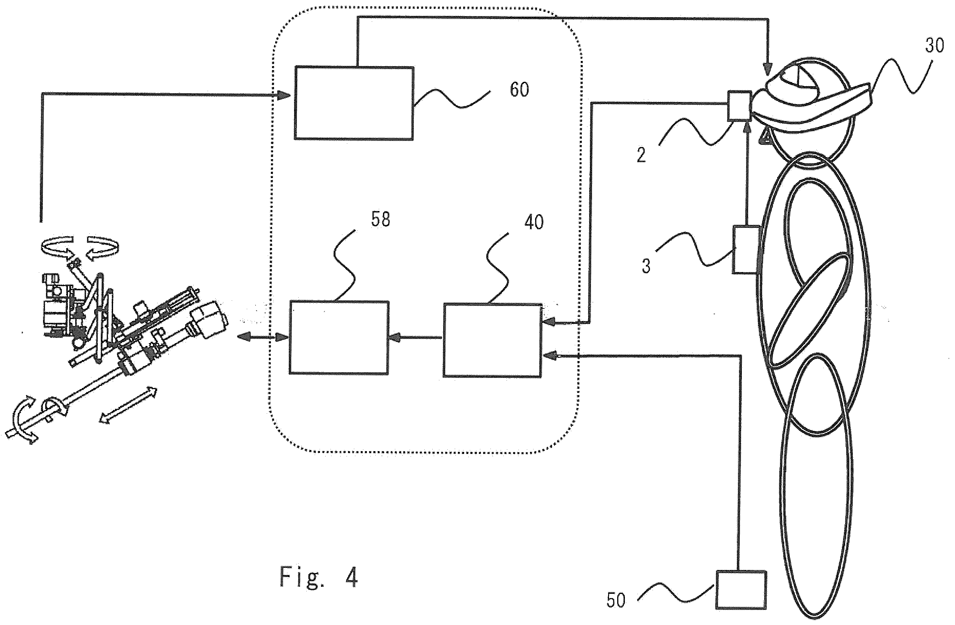

Further, in an example of an endoscope operation system according to this exemplary embodiment, the endoscope operation system includes gyroscopic sensors 2 and 3, a control unit 40, a bulb unit 58, an endoscope controller 60, and an on-off switching foot switch 50 as shown in Fig. 4. Further, the control unit 40 and the bulb unit 58 control the operation of the holding arm unit 10.

In an example of an endoscope operation system according to this exemplary embodiment, the endoscope 24 is attached to the holding arm unit 10 and an image taken (or imaged) by the endoscope 24 is displayed in the HMD 30. Further, in an example of an endoscope operation system according to this exemplary embodiment, a movement of the head of the surgery OP is detected by using the gyroscopic sensor 2 attached to the HMD 30 and the gyroscopic sensor 3 attached to the chest of the surgery OP, and the holding arm unit 10 is operated in a synchronized manner so as to follow the detected movement.

The gyroscopic sensor 2, which is an example of the first posture detection unit, is attached to the above-described HMD 30. The gyroscopic sensor 2 detects the above-described turning, left/right bending, (front/back) bending, and stretching in the HMD 30 and thereby detects an angular speed based on the postural displacement of the head of the surgery OP. A detection output from the gyroscopic sensor 2 is supplied to the later-described control unit 40. Note that the sensor that can be used as the first posture detection unit is not limited to the aforementioned sensor. For example, a magnetic sensor may be used as the first posture detection unit under an environment where the sensor hardly receive any influence of a magnetic field or an environment where there is no or little need to take the influence of a magnetic field into consideration. Further, a magnetic sensor or an acceleration sensor may be combined with the gyroscopic sensor in order to perform a zero point compensation of the gyroscopic sensor. Further, the place to which the gyroscopic sensor 2 is attached is not limited to the HMD 30. That is, the gyroscopic sensor 2 may be attached to other parts of the head of the surgery OP.

The gyroscopic sensor 3, which is an example of the second posture detection unit, is attached to the chest of the surgery OP. The gyroscopic sensor 3 detects an inclinational angular speed of the upper body of the surgery OP in the font/back and the left/right directions and thereby detects an angular speed based on the inclinational displacement of the upper body of the surgery OP. Note that the sensor that can be used as the second posture detection unit is not limited to the aforementioned sensor. For example, a magnetic sensor may be used as the second posture detection unit under an environment where the sensor hardly receive any influence of a magnetic field or an environment where there is no or little need to take the influence of a magnetic field into consideration. Further, a magnetic sensor or an acceleration sensor may be combined with the gyroscopic sensor in order to perform a zero point compensation of the gyroscopic sensor. Further, the place to which the gyroscopic sensor 3 is attached is not limited to the chest of the surgery OP. For example, the gyroscopic sensor 3 may be attached to the belly of the surgery OP. Further, the gyroscopic sensor 3 may be attached to any part of the torso of the surgery OP, provided that the attached gyroscopic sensor 3 can detect an inclinational angular speed of the torso of the surgery OP. A detection output from the gyroscopic sensor 3 is supplied to the later-described control unit 40. Further, the detection output that is supplied from each of the gyroscopic sensors 2 and 3 to the control unit 40 is supplied to the control unit 40 through, for example, CAN communication.

The endoscope controller 60 controls the operations of the zooming mechanism unit of the endoscope 24 and the light source based on a group of command signals supplied from the operation unit, and performs certain image processing based on picked-up image data DD obtained from a solid-state image pickup device of the endoscope 24. Further, the endoscope controller 60 creates image data by performing certain image processing based on picked-up image data and supplies the created image data to the control unit 40 and HMD 30. As a result, an image created based on the image data supplied from the endoscope controller 60 is displayed in the display unit(s) of the HMD 30 in a 3D manner.

The control unit 40 receives a signal group (i.e. a group of signals) GS1 representing an angular speed vector in each of the above-described directions of the head of the surgery OP supplied from the gyroscopic sensor 2 in the HMD 30, a signal group GS2 representing an angular speed vector in each of the above-described directions of the upper body of the surgery OP supplied from the gyroscopic sensor 3, and a command signal Cf representing an operation stop instruction for the holding arm unit 10 supplied from the on-off switching foot switch 50.

The control unit 40 includes a storage unit (not shown) that stores program data for the air-pressure control of the vane motor unit 16, the vane motor 20, the air-pressure cylinder 12, and the air-pressure cylinder 18, image data from the endoscope controller 60, data indicating a calculation result obtained by the control unit 40, and so on.

The control unit 40 creates control signals for controlling the vane motor unit 16, the vane motor 20, the air-pressure cylinder 12, and the air-pressure cylinder 18 in the aforementioned holding arm unit 10, and supplies the created control signals to the bulb unit 58. As a result, the bulb unit 58 controls respective valves based on the control signals supplied from the control unit 40, and supplies actuation air supplied from an air supply source to the vane motor unit 16, the vane motor 20, the air-pressure cylinder 12, and the air-pressure cylinder 18 in the holding arm unit 10.

The control unit 40 controls the amount and speed of the insertion of the insertion section of the endoscope 24 into the body of the subject, and makes the holding arm unit 10 operate so that the posture of the image pickup unit of the endoscope 24 is controlled.

The control unit 40 calculates the target speed value of the image pickup unit of the endoscope 24 based on the signal group GS1 representing the angular speed vector in each of the above-described directions of the head of the surgery OP supplied from the gyroscopic sensor 2 in the HMD 30 and the signal group GS2 representing the angular speed vector in each of the above-described directions of the upper body of the surgery OP supplied from the gyroscopic sensor 3. The control unit 40 creates control data based on the target speed value in order to operate the air-pressure cylinders 12 and 18 and the vane motor unit 16 of the holding arm unit 10 so that the image pickup unit of the endoscope 24 follows the target speed value, and the supplies the created control data to the bulb unit 58.

A method for setting a target speed value of the image pickup unit of the endoscope 24 performed by the control unit 40 is explained hereinafter in a more detailed manner.

Firstly, the control unit 40 calculates an angular speed command vector $\omega_{cmd}$ based on the signal group GS 1 representing the angular speed vector supplied from the gyroscopic sensor 2 by using the below-shown expression. That is, the control unit 40 calculates the angular speed command vector $\omega_{cmd}$ by multiplying an angular speed vector $\omega_{s1}$ of the head obtained from the gyroscopic sensor 2 by a predetermined coefficient matrix $K_r$.

[Expression 1]

$$\omega_{cmd} = K_r \cdot \omega_{s1} \quad \cdots (1)$$

The angular speed vector $\omega_{s1}$ is an angular speed vector of the head detected by the gyroscopic sensor 2 of the HMD 30. Note that as the coordinate system, a coordinate system fixed to the head is used. The central axis of the neck of the surgery OP shown in Fig. 2 is defined as a y-axis, and the left/right direction of the surgery OP is defined as an x-axis. Further, the front/back direction of the surgery OP is defined as a z-axis.

The angular speed vector $\omega_{s1}$ is a 3D vector including an inclinational angular speed of the head of the surgery OP in the front/back direction in the z-y axes plane in the coordinate system fixed to the head (an angular speed in a direction corresponding to a front/back rotation of the head with respect to the neck of the surgery OP), a rotational angular speed of the head of the surgery OP around the y-axis on the z-x axes plane (an angular speed in a direction corresponding to turning of the head of the surgery OP on the neck), and an inclinational angular speed of the head of the surgery OP in the left/right direction on the x-y axes plane (an angular speed in a direction corresponding to left/right bending of the head of the surgery OP).

[Expression 2]

$$\omega_{s1} = \left(\omega_{s1x}, \omega_{s1y}, \omega_{s1z}\right)^{t} \quad \cdots (2)$$

The coefficient matrix $K_r$ is a 3×3 diagonal matrix, in which predetermined coefficients representing speed gains are set in advance as diagonal components. A user can adjust the sensitivity according to his/her preference by multiplying the angular speed by the constant $K_r$. In this constant $K_r$, a different value can be set for each direction. The matrix $K_r$ may be a function.

[Expression 3]

$$K_r = \begin{bmatrix} K_{rx} & 0 & 0 \\ 0 & K_{ry} & 0 \\ 0 & 0 & K_{rz} \end{bmatrix} \quad \cdots (3)$$

Next, the control unit 40 sets the angular speed command vector $\omega_{cmd}$ to a predetermined limit value $\omega_{lim}$ by a limiter. That is, when the angular speed command vector $\omega_{cmd}$ exceeds the limit value $\omega_{lim}$, an angular speed command vector $\omega'_{cmd}$ is set to the limit value $\omega_{lim}$. Further, when the angular speed command vector $\omega_{cmd}$ is smaller than the limit value $\omega_{lim}$, the angular speed command vector $\omega'_{cmd}$ is set to the angular speed command vector $\omega_{cmd}$. This is done in order to prevent the holding arm unit 10 from operating at an excessive speed and thereby prevent the image pickup unit from damaging an internal organ. Note that data for the angular speed command vector $\omega'_{cmd}$ is stored in the storage unit.

Next, the control unit 40 converts the angular speed command vector $\omega'_{cmd}$ into local coordinates (Lx, Ly, Lz) of the holding arm unit 10 (see Fig. 3) according to the below-shown expression by using a transformation matrix T. Further, the control unit 40 multiplies the converted coordinates by a matrix R and thereby obtains an angular speed command vector $\omega''_{cmd}$ in an orthogonal coordinate system (Cx, Cy, Cz) at the tip of the endoscope 24 (see Fig. 3). The coordinate axis Cz in the orthogonal coordinate system is defined along the central axis line of the insertion section of the endoscope 24, i.e., along the forward-moving or the backward-moving direction of the image pickup unit of the endoscope 24.

[Expression 4]

$$\omega''_{cmd} = RT \cdot \omega'_{cmd} \quad \cdots (4)$$

[Expression 5]

$$R = E^{iq1} E^{jq2} E^{kq4} \quad \cdots (5)$$

The matrix R represents the posture of the endoscope 24 and is successively obtained from a joint displacement(s) q of the holding arm unit 10 (see $q_1$, $q_2$ and $q_4$ in Fig. 3) by forward kinematic calculation. In the expressions, E represents a rotating matrix.

In this way, the up/down and left/right directions on the screen of the display unit in the HMD 30 always conform to the up/down and left/right directions of the head of the surgery OP. That is, the coordinate system fixed to the tip of the endoscope 24 conforms to the coordinate system fixed to the head of the surgery OP in the HMD 30. Therefore, an image displayed in the display unit in the HMD 30 follows the movement of the head of the surgery OP.

Note that in the above-described example, the angular speed command vector $\omega'_{cmd}$ is converted into local coordinates (Lx, Ly, Lz) of the holding arm unit 10 by using the transformation matrix T, and then the angular speed command vector $\omega''_{cmd}$ in the orthogonal coordinate system (Cx, Cy, Cz) at the tip of the endoscope 24 is obtained by multiplying the converted coordinates by the matrix R. However, the present invention is not limited to such examples. The conversion from the local coordinates (Lx, Ly, Lz) of the holding arm unit 10 into the orthogonal coordinate system (Cx, Cy, Cz) at the tip of the endoscope 24 can be omitted. For example, in a case where an image displayed in the display unit of the HMD 30 is viewed as an external CRT image, the conversion from the local coordinates (Lx, Ly, Lz) of the holding arm unit 10 into the orthogonal coordinate system (Cx, Cy, Cz) at the tip of the endoscope 24 can be omitted so that the CRT image can be superimposed over a CT image.

Next, the control unit 40 converts the angular speed command vector $\omega''_{cmd}$ in the orthogonal coordinate system at the tip of the endoscope 24 into a target speed vector $v_{xy}$ of the tip (image pickup unit) of the endoscope 24 according to the below-shown expression. That is, the angular speed command vector $\omega''_{cmd}$ is converted into a component $v_{xy}$ in the up/down and left/right directions of the target speed of the tip of the endoscope 24 in the orthogonal coordinate system (Cx, Cy, Cz) by calculating the exterior product of the angular speed command vector $\omega''_{cmd}$ and a vector $l_3$ extending from the rotation center point GP of the holding arm unit 10 to the tip of the endoscope 24.

[Expression 6]

$$ v_{xy} = \omega''_{cmd} \times l_3 \quad \cdots (6) $$

Next, the control unit 40 performs calculation on the target speed vector $v_{xy}$ by using the below-shown expression in order to adjust the speed of the image pickup unit of the endoscope 24 so that the speed of the image pickup unit can be changed according to the insertion amount of the image pickup unit into the body. In this way, when the insertion amount in the forward direction of the image pickup unit of the endoscope 24 increases, a target speed vector $v'_{xy}$ of the image pickup unit of the endoscope 24 increases. On the other hand, when the insertion amount of the image pickup unit of the endoscope 24 decreases, i.e., when the image pickup unit of the endoscope 24 is pulled out from the body, the target speed vector $v'_{xy}$ of the image pickup unit of the endoscope 24 decreases.

[Expression 7]

$$ v'_{xy} = (1 + r_{xy} q_3) v_{xy} \quad \cdots (7) $$

The dependence of the moving amount on the screen on the insertion level is adjusted by multiplying the value of the target speed vector $v_{xy}$ by a coefficient $r_{xy}$ that is dependent on $q_3$ representing the insertion amount of the tip of the endoscope 24 (see Fig. 3). In this way, the amount of the movement in the visual field caused by a rotation of the head can be adjusted. For example, the amount of the movement of an object of interest on the screen that is caused when the head is rotated can be maintained substantially unchanged regardless of the zooming position. Therefore, the intuitiveness of the operation is improved.

Note that the coefficient $r_{xy}$ is a constant and defined in a range in which the polarity of the value of the target speed vector $v_{xy}$ is not reversed. However, it is assumed that the value $q_3$ is positive when the endoscope is inserted from the center position, and is negative when the endoscope is pulled from the center position. The center of the movable range of the value $q_3$ in Fig. 3 is defined as the center position, and the value q3 is zero at the center position. Note that the coefficient $r_{xy}$ may be a function.

Further, the control unit 40 calculates a target speed vector along the Cz-coordinate axis in the image pickup unit of the endoscope 24 (see Fig. 3) based on the signal group GS2 representing an angular speed vector supplied from the gyroscopic sensor 3. Therefore, the control unit 40 firstly calculates a speed vector $v_h$ at the neck position in the absolute coordinate system based on the signal group GS2 representing an angular speed vector supplied from the gyroscopic sensor 3. That is, an angular speed vector $\omega_{s2}$ of the upper body is converted into a speed command vector $v_h$ at the neck position in the absolute coordinate system by calculating the exterior product of the angular speed vector

$\omega_{s2}$ and a vector l'extending from the waist of the surgery OP to the neck position.
[Expression 8]

$$v_h = \omega_{s2} \times l' \quad \cdots (8)$$

In an example of an endoscope operation system according to this exemplary embodiment, the moving speed of the head in the front/back direction is regarded as a translational speed at the neck position in the front/back direction, and in reality, the moving speed of the head in the front/back direction is obtained as the moving speed of the neck part in the front/back direction.

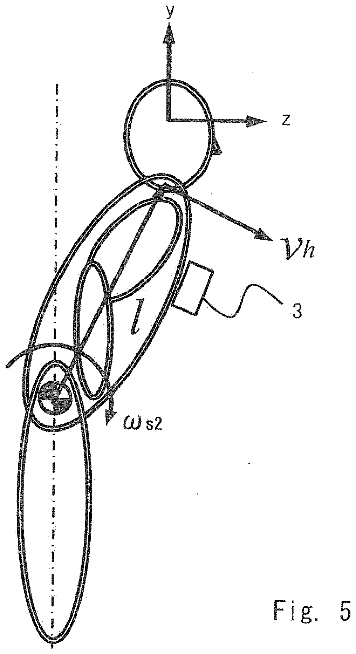

As shown in Fig. 5, the angular speed vector $\omega_{s2}$ represents a rotation speed caused by a rotational motion of the upper body around the waist, and is an angular speed vector of the upper body detected by the gyroscopic sensor 3 attached to the chest. Note that in the coordinate system, the vertically upward direction in Fig. 5 is defined as a y-axis and the right direction with respect to the surgery OP perpendicular to the y-axis is defined as an x-axis. Further, the forward direction with respect to the surgery OP perpendicular to the y- and x-axes is defined as a z-axis. In this absolute coordinate system, even when the upper body of the surgery OP is tilted, the y-axis of this coordinate system is always in the vertically upward direction in Fig. 5.

The angular speed vector $\omega_{s2}$ is a 3D vector including an inclinational angular speed of the upper body of the surgery OP in the front/back direction in the z-y axes plane in the above-described absolute coordinate system (an angular speed in a direction corresponding to tilting of the upper body of the surgery OP in the front/back direction), a rotational angular speed of the upper body of the surgery OP around the y-axis in the z-x axes plane, and an inclinational angular speed of the upper body of the surgery OP in the left/right direction in the x-y axes plane. Note that as described later, for a zooming operation in which the tip of the endoscope 24 is moved in the front/back direction, the inclinational angular speed component of the upper body of the surgery OP in the front/back direction of the components of the angular speed vector $\omega_{s2}$ is used.

Next, the control unit 40 converts the speed command vector $v_h$ at the neck position into a coordinate system fixed to the head of the surgery OP according to the below-shown expression by using a transformation matrix R' and thereby obtains a target speed vector $v_h$' at the neck positon. Note that as the coordinate system, a coordinate system fixed to the head is used. The central axis of the head of the surgery OP shown in Fig. 5 is defined as a y-axis, and the left/right direction of the surgery OP is defined as an x-axis. Further, the front/back direction of the surgery OP is defined as a z-axis.
[Expression 9]

$$v_h' = R' \cdot v_h \quad \cdots (9)$$

Note that when the inclination angle of the head is small and hence the transformation matrix R' is close to the unit matrix, the conversion calculation using Expression (9) may be omitted. Alternatively, the conversion calculation using Expression (9) may be performed only when precise calculation is necessary.

The target speed vector $v_h$' at the neck positon is a 3D vector including a moving speed of the head of the surgery OP in the up/down direction in the y-axis direction in the coordinate system fixed to the head, a moving speed of the head of the surgery OP in the front/back direction in the z-axis direction, and a moving speed of the head of the surgery OP in the left/right direction in the x-axis direction.

As described later, in an example of an endoscope operation system according to this exemplary embodiment, for a zooming operation in which the tip of the endoscope 24 is moved in the front/back direction, a command value for the zooming operation in which the tip of the endoscope 24 is moved in the front/back direction is calculated by using the moving speed of the head of the surgery OP in the front/back direction of the components of the target speed vector $v_h$' at the neck positon.

Note that the vector l' and the transformation matrix R' can be obtained from the inclination angles of the upper body and the head. The inclination angles of the upper body and the head can be obtained by, for example, integrating angular speeds obtained from the gyroscopic sensors 2 and 3, or obtained from outputs of a geomagnetic sensor or the like. Further, in actual operations, these inclination angles may be considered to be sufficiently small and hence may be approximated to zero. Even when they are defined as zero, no sense of wrongness or discomfort is caused in the operation.

Next, the control unit 40 converts the obtained target speed vector $v_h$' at the neck positon into a target speed vector $v_h$" of the tip (image pickup unit) of the endoscope 24 according to the below-shown expression. In this way, the control unit 40 can conform the forward/backward movement of the tip (image pickup unit) of the endoscope 24 to the forward/backward movement of the head. Note that the matrix R and the transformation matrix T are similar to those in the above-shown expressions.

[Expression 10]

$$v_h'' = RT \cdot v_h' \quad \cdots (10)$$

The control unit 40 obtains the final target speed value of the tip (image pickup unit) of the endoscope by using a component of the obtained target speed vector $v_h''$ of the tip (image pickup unit) of the endoscope 24 that corresponds to the moving speed of the head of the surgery OP in the front/back direction.

Note that additional processing such as further multiplying the component of the target speed vector $v_h''$ corresponding to the moving speed of the head of the surgery OP in the front/back direction by a predetermined coefficient may be performed.

Then, the control unit 40 adds "the target speed vector $v_{xy}$ of the tip (image pickup unit) of the endoscope 24 obtained based on the gyroscopic sensor 2" and "the target speed vector $v_h''$ of the tip (image pickup unit) of the endoscope 24 obtained based on the gyroscopic sensor 3". It should be noted that for the target speed vector $v_{xy}''$, only "the component corresponding to the moving speed of the head of the surgery OP in the front/back direction" is added. In this way, the control unit 40 calculates the final target speed value of the tip (image pickup unit) of the endoscope 24 by adding the speed components in the up/down and left/right directions, and in the front/back direction.

Note that in the above-described example, for the roll component of the rotation speed of the head of the surgery (the neck tilting action), the roll component of the above-described angular speed command vector $\omega'_{cmd}$ is directly used as the target speed of the roll $q_4$ of the endoscope. However, the present invention is not limited to such examples. Further, this operation may be disabled (or omitted).

The advantageous effects that are achieved by using the on-off switching foot switch 50 include the following ones. When the surgery OP does not want to move the endoscope 24, the surgery OP may turn off the switch so that he/she can freely move his/her head and upper body. Further, for example, when the surgery OP moves the endoscope 24 to the right while the switch is in an on-state but his/her head reaches the limit of its movable range, the surgery OP can move the endoscope 24 even further by turning off the switch and retuning his/her head to the left, and then turning on the switch and moving his/her head to the right. Further, since the endoscope 24 does not move in conjunction with the movement of the head unless the switch is turned on, unexpected movements of the endoscope 24 can be prevented.

As explained above, according to an example of an endoscope operation system in accordance with this exemplary embodiment, the gyroscopic sensor 3 is attached to the chest in addition to the gyroscopic sensor 2 attached to the head; the movement of the head is detected by the gyroscopic sensor 2 and in addition to that, the speed of the head in the front/back direction is obtained from the tilting of the upper body in the front/back direction detected by the gyroscopic sensor 3; a target speed value of the tip of the endoscope 24 for carrying out a movement of the endoscope 24 in the up/down and left/right directions, a rotation movement of the endoscope 24 around its axis, and a movement of the endoscope 24 in the front/back direction is calculated based on the aforementioned movement information; and an instruction is provided to the holding arm unit 10. In this way, it is possible to easily and intuitively carry out an operation of the visual field of the endoscope 24.

Further, according to an example of an endoscope operation system in accordance with this exemplary embodiment, since the degrees of freedom of movements that the surgery OP can carry out without using his/her hands increase in the operation of the visual field, the need for the input device necessary in the related art such as a foot switch for a zooming operation, for example, can be eliminated. Further, since a translational operation of the visual field to follow the movement of the head can be carried out, the intuitiveness of the operation dramatically improves and the risk of operation errors can be reduced.

Therefore, according to an example of an endoscope operation system in accordance with this exemplary embodiment, the following advantageous effects can be provided.

1. In view of signal shut-off, noises, and setting, there is no need to install an external sensor.
2. All the operations can be performed without using hands.
3. Operations can be intuitively performed without thinking about the operation procedure.
4. The use of the foot switch is minimized.

That is, according to an example of an endoscope operation system in accordance with this exemplary embodiment, it is possible to easily and intuitively carry out a translational operation method of the visual field such as a zooming operation while achieving all of the above-described advantageous effects.

Second exemplary embodiment

According to an example of an endoscope operation system in accordance with the above-described first exemplary embodiment, the tip of the endoscope 24 shown in Fig. 3 is always in an extended state and is not configured to be bent. In contrast to this, according to an example of an endoscope operation system in accordance with this exemplary embodiment, the endoscope 24 is configured so that at least a part of the tip of the endoscope 24 can be bent. That is, at least a part of the tip of the endoscope 24 can be bent in the left/right direction with respect to the rotation axis line G.

The holding arm unit 10 can also carry out a movement of anther one degree of freedom (a movement in the left/right direction to follow the left/right movement of the upper body of the surgery OP) in addition to the four degrees of freedom of movements of the image pickup unit of the endoscope 24 (movements in the up/down, left/right, rotational, and front/back directions to follow the movements of the head and the upper body of the surgery OP). That is, the holding arm unit 10 is configured to support the tip of the endoscope 24 in such a manner that the tip of the endoscope 24 can be moved in up/down, left/right, and front/back directions, and rotated on its own axis. Further, the holding arm unit 10 is configured to support the tip of the endoscope 24 so that at least a part of the tip of the endoscope 24 can be bent in the left/right direction.

The control unit 40 can detect the inclinational angular speed of the upper body of the surgery OP in the left/right direction by using the gyroscopic sensor 3 attached to the upper body on a similar principle to the above-described principle in the first exemplary embodiment. Then, the control unit 40 can calculate the moving speed of the neck positon of the surgery OP in the left/right direction from this detected inclinational angular speed of the upper body of the surgery OP in the left/right direction. Further, the control unit 40 can calculate the moving speed of the head of the surgery OP in the left/right direction from this calculated moving speed of the neck positon in the left/right direction. The control unit 40 uses this calculated moving speed of the head of the surgery OP in the left/right direction for an operation command for bending at least a part of the tip of the endoscope 24. For example, when the surgery OP tilts to the right, the control unit 40 bends at least a part of the tip of the endoscope 24 to the right to follow this movement. Further, when the surgery OP tilts to the left, the control unit 40 bends at least a part of the tip of the endoscope 24 to the left to follow this movement.

As described above, according to an example of an endoscope operation system in accordance with this exemplary embodiment, for the endoscope 24 capable of bending at least a part of its tip, it is possible to bend at least the part of the tip of the endoscope 24 in the left/right direction and move the tip itself in a translational manner as explained in the first exemplary embodiment. In the above-described endoscope 24 capable of bending at least a part of its tip, it is possible to bend only the part of the tip of the endoscope 24 and thereby point the tip to the left or right, instead of moving the entire tip of the endoscope 24 in order to move the tip of the endoscope 24 to the left or right.

Third exemplary embodiment

According to an example of an endoscope operation system in accordance with the above-described second exemplary embodiment, at least a part of the tip of the endoscope 24 can be bent in the left/right direction with respect to the rotation axis line G. According to an example of an endoscope operation system in accordance with this exemplary embodiment, at least a part of the tip of the endoscope 24 can be bent in the up/down direction as well as in the left/right direction.

The holding arm unit 10 can also carry out a movement of anther two degrees of freedom (a movement in the left/right direction to follow the left/right movement of the upper body of the surgery OP, and a movement in the up/down direction to follow the movement of the whole head of the surgery OP in the vertically up/down direction) in addition to the four degrees of freedom of movements of the image pickup unit of the endoscope 24 (movements in the up/down, left/right, rotational, and front/back directions to follow the movements of the head and the upper body of the surgery OP). That is, the holding arm unit 10 is configured to support the tip of the endoscope 24 in such a manner that the tip of the endoscope 24 can be moved in up/down, left/right, and front/back directions, and rotated on its own axis. Further, the holding arm unit 10 is configured to support the tip of the endoscope 24 so that at least a part of the tip of the endoscope 24 can be bent in the left/right direction and in the up/down directions.

In an example of an endoscope operation system according to this exemplary embodiment, the moving speed of the whole head in the vertically up/down direction is regarded as the translational speed of the neck position in the up/down direction, and in reality, the moving speed of the whole head in the vertically up/down direction is obtained as the moving speed of the neck part in the up/down direction. The moving speed of the neck positon in the up/down direction can be obtained from, for example, the translational speed of the waist position in the up/down direction. Further, the translational speed of the waist position in the up/down direction can be obtained from, for example, the rotational angular speed of the waist by the bending and stretching motion of a knee(s) of the surgery OP.

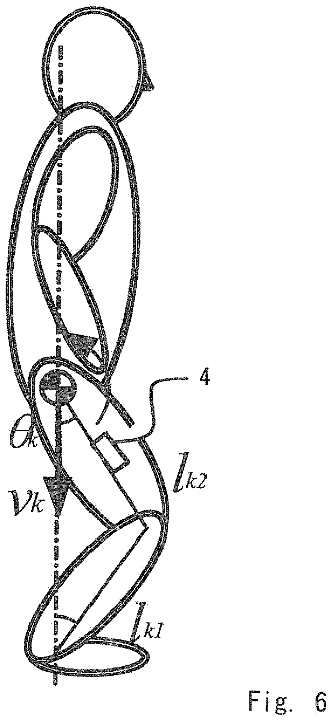

For example, as shown in Fig. 6, a gyroscopic sensor 4, which is an example of the third posture detection unit, is attached to a knee of the surgery OP. The gyroscopic sensor 4 detects the rotational angular speed of the waist based on the bending and stretching motion of the knee of the surgery OP. Note that the place where the gyroscopic sensor 4 is attached is not limited to the knees of the surgery OP. That is, the gyroscopic sensor 4 may be attached to any part of the the thigh or the lower thigh of the surgery OP, provided that the attached gyroscopic sensor 4 can detect the rotational angular speed of the waist of the surgery OP. A detection output from the gyroscopic sensor 4 is supplied to the above-described control unit 40.

The control unit 40 approximately calculates a component $v_{ky}$ in the vertical direction of a translational speed $v_k$ of the waist position from a rotational angular speed $\dot{\theta}_k$ (in the expression, a variable with a dot added thereon) of the waist in the knee bending and stretching motion detected by the gyroscopic sensor 4. Note that the other components of the translational speed vector $v_k$ of the waist position may be defined as zero.

[Expression 11]

$$v_{ky} = (l_{k1} + l_{k2})(1 - \cos\theta_k)\dot{\theta}_k \cdots (11)$$

Note that the rotational angle $\theta_k$ of the knee is a rotational angular speed of the waist by the knee bending and stretching motion in the vertically upward direction, and becomes 0° when the surgery OP is in an upright posture. Further, $l_{k1}$ represents the length from the ankle to the knee and $l_{k2}$ represents the length from the knee to the waist.

Note that a component $v_{ky}$ in the vertical direction of a translational speed $v_k$ of the waist position in Expression (11) may be calculated by multiplying the rotational angular speed $\dot{\theta}_k$ by a constant value K as shown in the below-shown expression.

[Expression 12]

$$v_{ky} = K\dot{\theta}_k \cdots (12)$$

The above-shown Expression (9) is replaced by the below-shown expression by adding the above-shown Expression (12) to the speed $v_h$ of the neck positon in the above-shown Expression (8).

[Expression 13]

$$v_h' = R' \cdot (v_h + v_k) \quad \cdots (13)$$

As described above, the control unit 40 uses this calculated moving speed of the whole head of the surgery OP in the vertically up/down direction for an operation command for bending at least a part of the tip of the endoscope 24. For example, when the surgery OP bends his/her knee(s) by a bending and stretching motion, the control unit 40 bends at least a part of the tip of the endoscope 24 downward to follow this movement of the surgery OP. Further, when the surgery OP extends his/her knee(s) by a bending and stretching motion, the control unit 40 bends at least a part of the tip of the endoscope 24 upward to follow this movement of the surgery OP.

As described above, according to an example of an endoscope operation system in accordance with this exemplary embodiment, it is possible to detect a vertically up/down motion of the whole head independent of the tilting of the upper body. Further, the control unit 40 bends at least a part of the tip of the endoscope 24 in the up/down direction in accordance with the detected value of this motion. As a result, it is possible to carry out six degrees of freedom of movements in total for the movements of the tip of the endoscope 24.

Other exemplary embodiments

In the above-described first exemplary embodiment, a case where a gyroscopic sensor is used as the second posture detection unit is explained. However, the present invention is not limited to such cases. A compact camera that is attached to the front of the torso may be used as the second posture detection unit. When a compact camera is used, the inclinational angular speed of the upper body in the front/back direction can be estimated based on an optical flow of an image in the forward direction of the surgery OP taken (or imaged) by the compact camera.

The surgery OP, who is wearing the HMD 30, cannot view the forward direction. However, there is an advantage that the surgery OP can observe the scene in the forward direction, for example, by displaying a part of the forward image taken by the compact camera in a sub-monitor of the HMD 30 or switching the image displayed in the display unit of the HMD 30 to the forward image.

Note that the present invention is not limited to the above-described exemplary embodiments, and needless to say, various modifications can be made without departing from the spirit and scope of the present invention. For example, although examples where a gyroscopic sensor or a compact camera is used as the second posture detection unit are explained, the present invention is not limited to such examples. For example, a magnetic sensor may be used as the second posture detection unit under an environment where the sensor hardly receive any influence of a magnetic field or an environment where there is no or little need to take the influence of a magnetic field into consideration.

Further, the specific configurations of the holding arm unit 10 and the endoscope 24 are not limited to the above-described exemplary embodiments. That is, a holding arm unit 10 and an endoscope 24 having other configurations may be used.

Further, the above explanations are given on the assumption that images displayed in the display unit, which displays an image based on an image signal supplied from the image pickup unit of the endoscope 24, are displayed by the HMD 30. However, the present invention is not limited to such configurations. For example, the display unit may display images by using well-known display means such as a typical liquid crystal monitor.

Although the present invention has been explained with reference to certain embodiments, the present invention is not limited to those embodiments. Various modifications can be made to the configurations and the details of those embodiments by those skilled in the art without departing from the scope of the present invention.

**Reference Signs List**

| | |
|---|---|
| 2, 3, 4 | GYROSCOPIC SENSOR |
| 10 | HOLDING ARM UNIT |
| 12 | AIR-PRESSURE CYLINDER |
| 14 | PARALLEL LINKAGE |
| 16 | VANE MOTOR UNIT |
| 18 | AIR-PRESSURE CYLINDER |
| 20 | VANE MOTOR |
| 22 | TIMING BELT |
| 24 | ENDOSCOPE |
| 30 | HMD |
| 40 | CONTROL UNIT |
| 50 | ON-OFF SWITCHING FOOT SWITCH |
| 58 | BULB UNIT |
| 60 | ENDOSCOPE CONTROLLER |

**Claims**

1. An endoscope operation system comprising:

    an endoscope comprising an image pickup unit at its tip;
    a holding arm unit that supports the tip of the endoscope in such a manner that the tip of the endoscope can be moved in up/down, left/right, and front/back directions, and can be rotated on its own axis;
    a display unit that displays an image based on an image signal supplied from the image pickup unit of the endoscope;
    a first posture detection unit configured to be attached to a head of an operator, the first posture detection unit being configured to detect an angular speed based on a postural displacement of the head of the operator; and
    **characterized by**
    a second posture detection unit configured to be attached to a torso of the operator, the second posture detection unit being configured to detect an angular speed based on an inclinational displacement of an upper body of the operator; and
    a control unit that calculates a target speed vector at the tip of the endoscope and controls a moving direction and a speed of the tip of the endoscope so that the tip of the endoscope follows the calculated target speed vector, wherein
    the control unit calculates a translational speed of a neck position of the operator in the front/back direction based on an inclinational angular speed component of the upper body in the front/back direction of the angular speed calculated based on the inclinational displacement of the upper body of the operator detected by the second posture detection unit, and calculates the target speed vector based on the calculated translational speed of the neck position of the operator in the front/back direction and information of a movement of a posture of the head of the operator detected by the first posture detection unit.

2. The endoscope operation system according to Claim 1, wherein the control unit calculates a translational speed of the head of the operator in the front/back direction based on the calculated translational speed of the neck positon of the operator in the front/back direction, and calculates the target speed vector based on the calculated translational speed of the head of the operator in the front/back direction and the information of the movement of the posture of the head of the operator detected by the first posture detection unit.

3. The endoscope operation system according to Claim 1 or 2, wherein
   at least a part of the tip of the endoscope can be bent in a left/right direction,
   the holding arm unit supports the tip of the endoscope in such a manner that the tip of the endoscope can be moved in up/down, left/right, and front/back directions, and can be rotated on its own axis, and at least the part of the tip of the endoscope can be bent in the left/right direction, and
   the control unit calculates a translational speed of the neck position of the operator in the left/right direction based on an inclinational angular speed component of the upper body in the left/right direction of the angular speed calculated based on the inclinational displacement of the upper body of the operator detected by the second posture detection unit, and calculates the target speed vector based on the calculated translational speed of the neck position of the operator in the left/right direction and the information of the movement of the posture of the head of the operator detected by the first posture detection unit.

4. The endoscope operation system according to Claim 3, further comprising a third posture detection unit attached to a thigh or a lower thigh of the operator, the third posture detection unit being configured to detect an angular speed of a waist based on a bending and stretching motion of a knee of the operator, wherein
   at least a part of the tip of the endoscope can be bent in a up/down direction,
   the holding arm unit supports the tip of the endoscope in such a manner that the tip of the endoscope can be moved in up/down, left/right, and front/back directions, and can be rotated on its own axis, and at least a part of the tip of the endoscope can be bent in the left/right direction and in the up/down direction, and
   the control unit calculates a translational speed of a waist position in a vertical direction based on the angular speed of the waist calculated based on the bending and stretching motion of the knee of the operator detected by the third posture detection unit, and calculates the target speed vector based on the calculated translational speed of the waist position in the vertical direction, the calculated translational speed of the neck position of the operator in the left/right direction, and the information of the movement of the posture of the head of the operator detected by the first posture detection unit.

5. The endoscope operation system according to any one of Claims 1 to 4, wherein the second posture detection unit is a gyroscopic sensor that detects an angular speed based on an inclinational displacement of the upper body of the operator.

6. The endoscope operation system according to any one of Claims 1 to 4, wherein the second posture detection unit is a camera attached to a front of the torso of the operator, and an inclinational angular speed of the upper body in the front/back direction is estimated based on an optical flow of an image in a forward direction of the operator taken by the camera.

**Patentansprüche**

1. Endoskopbetriebssystem umfassend:

   ein Endoskop , mit einer Bildaufnahmeeeinheit an seiner Spitze;
   einen Haltearm, der die Spitze des Endoskops in einer Art und Weise stützt, dass sie nach oben/unten, links/rechts und Vorwärts-/Rückwärts-Richtung bewegt und um ihre eigene Achse gedreht werden kann;
   eine Anzeigeeinheit, die ein Bild auf Basis eines Bildsignals von der Bildaufnahmeeeinheit des Endoskops anzeigt;
   einen ersten Gyrosensor, der dazu konfiguriert ist, um mit dem Kopf einer Bedienperson verbunden zu werden, wobei der erste Gyrosensor dazu konfiguriert ist, um eine Winkelgeschwindigkeit auf Basis einer Haltungsverlagerung des Kopfes der Bedienperson zu erkennen und **gekennzeichnet durch**

einen zweiten Gyrosensor, der dazu konfiguriert ist, um mit dem Torso einer Bedienperson verbunden zu werden, wobei der zweite Gyrosensor dazu konfiguriert ist, um eine Winkelgeschwindigkeit auf Basis einer Neigungsänderung des Oberkörpers der Bedienperson zu erkennen; und

eine Steuereinheit, die einen Sollgeschwindigkeitsvektor an der Spitze des Endoskops berechnet und die Bewegungsrichtung und -geschwindigkeit der Spitze des Endoskops steuert, so dass die Spitze des Endoskops dem berechneten Sollgeschwindigkeitsvektor folgt, wobei

die Steuereinheit die Translationsgeschwindigkeit einer Halsposition der Bedienperson, auf Basis einer Neigungswinkel-Geschwindigkeitskomponente des Oberkörpers der Bedienperson in Vorwärts-/Rückwärts-Richtung der berechneten Winkelgeschwindigkeit, auf Basis der vom zweiten Gyrosensor erkannten Neigungsveränderung des Oberkörpers der Bedienperson berechnet, und den Sollgeschwindigkeitsvektor auf Basis der berechneten Translationsgeschwindigkeit der Halsposition der Bedienperson in Vorwärts-/Rückwärts-Richtung und der Information über die **durch** den ersten Gyrosensor erkannte Bewegung der Kopfhaltung der Bedienperson berechnet.

2. Endoskopbetriebssystem gemäß Anspruch 1, wobei die Steuereinheit die Translationsgeschwindigkeit der Halsposition der Bedienperson, in Vorwärts-/Rückwärts-Richtung, auf Basis einer berechneten Translationsgeschwindigkeit der Halsposition der Bedienperson in Vorwärts-/Rückwärts-Richtung berechnet und den Sollgeschwindigkeitsvektor berechnet auf Basis der berechneten Translationsgeschwindigkeit des Kopfes der Bedienperson in Vorwärts-/Rückwärts-Richtung und der Information über die durch den ersten Gyrosensor erkannte Bewegung der Kopfhaltung der Bedienperson.

3. Endoskopbetriebssystem gemäß Anspruch 1 oder 2, wobei
mindestens ein Teil der Endoskopspitze in eine Links-/Rechtsrichtung gebogen werden kann,
der Haltearm die Spitze des Endoskops in einer Art und Weise stützt, dass sie nach oben/unten, links/rechts und vor/zurück-Richtung bewegt und um ihre eigene Achse gedreht werden kann; und mindestens ein Teil der Endoskopspitze in Links-/Rechtsrichtung gebogen werden kann, und
die Steuereinheit die Translationsgeschwindigkeit der Halsposition der Bedienperson in Links-/Rechts-Richtung, auf Basis einer Neigungswinkelgeschwindigkeitskomponente des Oberkörpers in Links-/Rechts-Richtung der berechneten Winkelgeschwindigkeit, die auf Basis der vom zweiten Gyrosensor erkannten Neigungsveränderung des Oberkörpers der Bedienperson berechnet wird, berechnet, und den Sollgeschwindigkeitsvektor auf Basis der berechneten Translationsgeschwindigkeit der Halsposition der Bedienperson in Links-/Rechts-Richtung und der Information über die durch den ersten Gyrosensor erkannte Bewegung der Kopfhaltung der Bedienperson berechnet.

4. Endoskopbetriebssystem gemäß Anspruch 3, das weiterhin einen dritten Gyrosensor umfasst, der an einem Ober- oder Unterschenkel der Bedienperson befestigt ist, wobei der dritte Gyrosensor dazu konfiguriert ist, um die Winkelgeschwindigkeit eines Unterkörpers auf Basis einer Beuge- und Streckbewegung eines Knies der Bedienperson zu erkennen, wobei
mindestens ein Teil der Endoskopspitze in eine Oben-/Unten-Richtung gebogen werden kann,
der Haltearm die Spitze des Endoskops in einer Art und Weise stützt, dass sie nach oben/unten, links/rechts und vor/zurück-Richtung bewegt und um ihre eigene Achse gedreht werden kann; und mindestens ein Teil der Endoskopspitze in Links/Rechts-Richtung und Oben-/Unten-Richtung gebogen werden kann, und
die Steuereinheit die Translationsgeschwindigkeit der Unterkörperposition in vertikaler Richtung auf Basis der Winkelgeschwindigkeit des Unterkörpers, die auf Basis der vom dritten Gyrosensor erkannten Beuge- und Streckbewegung des Knies der Bedienperson berechnet wird, berechnet, und den Sollgeschwindigkeitsvektor berechnet auf Basis der berechneten Translationsgeschwindigkeit der Unterkörperposition in vertikaler Richtung, die berechnete Translationsgeschwindigkeit der Halsposition der Bedienperson in Links-/Rechts-Richtung und der Information über die durch den ersten Gyrosensor erkannte Bewegung der Kopfhaltung der Bedienperson.

5. Endoskopbetriebssystem gemäß einem der Ansprüche 1 bis 4, wobei es sich beim zweiten Gyrosensor um einen gyroskopischen Sensor handelt, der eine Winkelgeschwindigkeit auf Basis einer Neigungsveränderung des Oberkörpers der Bedienperson erkennt.

6. Endoskopbetriebssystem gemäß einem der Ansprüche 1 bis 4, wobei es sich beim zweiten Gyrosensor um eine Kamera handelt, die an der Vorderseite des Torsos der Bedienperson befestigt ist, und eine Neigungswinkelgeschwindigkeit des Oberkörpers in Vorwärts-/Rückwärts-Richtung auf Basis des optischen Flusses eines Bildes in Vorwärtsrichtung der Bedienperson geschätzt wird, das von der Kamera aufgenommen wird.

**Revendications**

1. Système de fonctionnement d'un endoscope comprenant :

    un endoscope comprenant une unité de capture d'image à sa pointe ;
    une unité bras de maintien qui supporte la pointe de l'endoscope d'une manière telle que la pointe de l'endoscope peut être déplacée dans les directions haut/bas, gauche/droite et avant/arrière, et peut être tournée sur son propre axe ;
    une unité d'affichage qui affiche une image sur la base d'un signal d'image apporté depuis l'unité de capture d'image de l'endoscope ;
    une première unité de détection de position configurée pour être attachée à la tête d'un opérateur, la première unité de détection de position étant configurée pour détecter une vitesse angulaire sur la base d'un déplacement de position de la tête de l'opérateur ; et **caractérisé par**
    une deuxième unité de détection de position configurée pour être attachée au torse de l'opérateur, la deuxième unité de détection de position étant configurée pour détecter une vitesse angulaire sur la base d'un déplacement d'inclinaison de la partie supérieure du corps de l'opérateur ; et
    une unité de commande qui calcule un vecteur de vitesse cible à la pointe de l'endoscope et commande une direction de mouvement et une vitesse de la pointe de l'endoscope de telle sorte que la pointe de l'endoscope suit le vecteur de vitesse cible calculé, dans lequel
    l'unité de commande calcule une vitesse de translation d'une position de cou de l'opérateur dans la direction avant/arrière sur la base d'une composante de vitesse angulaire d'inclinaison de la partie supérieure du corps dans la direction avant/arrière de la vitesse angulaire calculée sur la base du déplacement d'inclinaison de la partie supérieure du corps de l'opérateur détecté par la deuxième unité de détection de position, et calcule le vecteur de vitesse cible sur la base de la vitesse de translation calculée de la position de cou de l'opérateur dans la direction avant/arrière et d'informations d'un déplacement d'une position de la tête de l'opérateur détecté par la première unité de détection de position.

2. Système de fonctionnement d'un endoscope selon la revendication 1, dans lequel l'unité de commande calcule une vitesse de translation de la tête de l'opérateur dans la direction avant/arrière sur la base de la vitesse de translation calculée de la position de cou de l'opérateur dans la direction avant/arrière, et calcule le vecteur de vitesse cible sur la base de la vitesse de translation calculée de la tête de l'opérateur dans la direction avant/arrière et des informations du déplacement de la position de la tête de l'opérateur détecté par la première unité de détection de position.

3. Système de fonctionnement d'un endoscope selon la revendication 1 ou 2, dans lequel
    au moins une partie de la pointe de l'endoscope peut être pliée dans une direction gauche/droite,
    l'unité bras de maintien supporte la pointe de l'endoscope d'une manière telle que la pointe de l'endoscope peut être déplacée dans les directions haut/bas, gauche/droite et avant/arrière, et peut être tournée sur son propre axe, et au moins une partie de la pointe de l'endoscope peut être pliée dans la direction gauche/droite, et
    l'unité de commande calcule une vitesse de translation de la position de cou de l'opérateur dans la direction gauche/droite sur la base d'une composante de vitesse angulaire d'inclinaison de la partie supérieure du corps dans la direction gauche/droite de la vitesse angulaire calculée sur la base du déplacement d'inclinaison de la partie supérieure du corps de l'opérateur détecté par la deuxième unité de détection de position, et calcule le vecteur de vitesse cible sur la base de la vitesse de translation calculée de la position de cou de l'opérateur dans la direction gauche/droite et des informations du déplacement de la position de la tête de l'opérateur détecté par la première unité de détection de position.

4. Système de fonctionnement d'un endoscope selon la revendication 3, comprenant en outre une troisième unité de détection de position attachée à une cuisse ou à la partie inférieure d'une cuisse de l'opérateur, la troisième unité de détection de position étant configurée pour détecter une vitesse angulaire d'une taille sur la base d'un mouvement de pliage et d'étirement d'un genou de l'opérateur, dans lequel
    au moins une partie de la pointe de l'endoscope peut être pliée dans une direction haut/bas,
    l'unité bras de maintien supporte la pointe de l'endoscope d'une manière telle que la pointe de l'endoscope peut être déplacée dans les directions haut/bas, gauche/droite et avant/arrière, et peut être tournée sur son propre axe, et au moins une partie de la pointe de l'endoscope peut être pliée dans la direction gauche/droite et dans la direction haut/bas, et
    l'unité de commande calcule une vitesse de translation d'une position de taille dans une direction verticale sur la base de la vitesse angulaire de la taille calculée sur la base du mouvement de pliage et d'étirement du genou de l'opérateur détecté par la troisième unité de détection de position, et calcule le vecteur de vitesse cible sur la base de la vitesse de translation calculée de la position de taille dans la direction verticale, de la vitesse de translation calculée de la position de cou de l'opérateur dans la direction gauche/droite et des informations du déplacement de la position de la tête de l'opérateur détecté par la première unité de détection de position.

5. Système de fonctionnement d'un endoscope selon l'une quelconque des revendications 1 à 4, dans lequel la deuxième unité de détection de position est un capteur gyroscopique qui détecte une vitesse angulaire sur la base d'un déplacement d'inclinaison de la partie supérieure du corps de l'opérateur.

6. Système de fonctionnement d'un endoscope selon l'une quelconque des revendications 1 à 4, dans lequel la deuxième unité de détection de position est une caméra attachée à une partie avant du torse de l'opérateur, et une vitesse angulaire d'inclinaison de la partie supérieure du corps dans la direction avant/arrière est estimée sur la base d'un flux optique d'une image dans une direction vers l'avant de l'opérateur prise par la caméra.

Fig. 1

Fig. 2

EP 2 918 216 B1

Fig. 3

Fig. 4

Fig. 5

Fig. 6

**EP 2 918 216 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10309258 B

- US 2011234484 A

**Non-patent literature cited in the description**

- Naviot. Medical endoscope grasping device. Hitachi Hybrid Network Co., Ltd,